# EUROPÄISCHE PATENTSCHRIFT

(11) **EP 1 781 603 B1**
(45) Veröffentlichungstag und Bekanntmachung des Hinweises auf die Patenterteilung: **06.06.2012**
(21) Anmeldenummer: 05758847.7
(22) Anmeldetag: 12.07.2005
(51) Int. Cl.: C07C 319/20, C07C 323/52

(54) **HERSTELLUNG VON 2-HYDROXY-4-METHYLTHIOBUTTERSÄURE**
PRODUCTION OF 2-HYDROXY-4-METHYLTHIOBUTYRIC ACID
PRODUCTION D'ACIDE BUTYRIQUE 2-HYDROXY-4-METHYLTHIO

(30) Priorität: 26.08.2004 DE 102004041250
(43) Veröffentlichungstag der Anmeldung: 09.05.2007
(73) Patentinhaber: Evonik Degussa GmbH, 45128 Essen (DE)
(72) Erfinder: MÖLLER, Alexander, 63571 Gelnhausen (DE); HASSEBERG, Hans-Albrecht, 63584 Gründau-Lieblos (DE); HEINZEL, Harald, 63674 Altenstadt (DE); HÄFNER, Volker, 63505 Langenselbold (DE)
(86) Internationale Anmeldenummer: PCT/EP2005/007530
(87) Internationale Veröffentlichungsnummer: WO 2006/021268

(56) Entgegenhaltungen:
- WO-A-96/40630
- KIRCHHEINER R ET AL: "NICROFER 5923 HMO, EIN NEUER HOCHKORROSIONSBESTAENDIGER WERKSTOFF FUER DIE CHEMISCHE INDUSTRIE, DIE UMWELTTECHNIK UND VERWANDTE ANWENDUNGEN" WERKSTOFFE UND KORROSION, VERLAG CHEMIE GMBM. WEINHEIM, DE, Bd. 43, Nr. 8, 1. August 1992 (1992-08-01), Seiten 388-395, XP000349761 ISSN: 0947-5117
- KOHLER M ET AL: "NICROFER 2509 SI 7, EIN NEUER KORROSIONSBESTANDIGER WERKSTOFF FUR DIE HANDHABUNG HEISSER, HOCHKONZENTRIERTER MINERALSAUREN ALLOY 700 SI, A NEW CORROSION RESISTANT MATERIAL FOR HANDLING OF HOT, HIGHLY CONCENTRATED MINERAL ACIDS" WERKSTOFFE UND KORROSION, VERLAG CHEMIE GMBM. WEINHEIM, DE, Bd. 46, Nr. 1, Januar 1995 (1995-01), Seiten 18-26, XP000494679 ISSN: 0947-5117
- DATABASE CAPLUS [Online] CHEMICAL ABSTRACTS SERVICE, COLUMBUS, OHIO, US; SUDO, SHOJI ET AL: "Preparation of methionines" XP002352909 gefunden im STN Database accession no. 2003:271701 & JP 2003 104959 A2 (NIPPON SODA CO., LTD., JAPAN) 9. April 2003 (2003-04-09)

## Beschreibung

Die vorliegende Erfindung bezieht sich auf einen Prozess zur Herstellung von 2-Hydroxy-4-methylthiobuttersäure, die den Einsatz von speziellen Werkstoffen für die unterschiedlichen Verfahrensstufen beschreibt.

### Stand der Technik

Nutritivitätsverbessernde Futtermittelzusatzstoffe sind heute ein unverzichtbarer Bestandteil der Tierernährung. Sie dienen der besseren Verwertung des Nahrungsangebotes, stimulieren das Wachstum und fördern die Eiweißbildung. Einer der wichtigsten dieser zusatzstoffe ist die essentielle Aminosäure Methionin, die vor allem in der Geflügelaufzucht als Futtermitteladditiv eine herausragende Stellung einnimmt. Auf diesem Gebiet haben, aber auch sogenannte Methionin-Ersatzstoffe wie das Methionin-Hydroxy-Analog (abgekürzt MHA) nicht unerhebliche Bedeutung, da sie ähnliche wachstumsstimulierende Eigenschaften aufweisen wie die dafür bekannte Aminosäure.

Die racemische Form der 2-Hydroxy-4-methylthio-buttersäure ist ein seit langem bekannter Methionin-Ersatzstoff, der hauptsächlich in der Tierernährung, insbesondere bei der Aufzucht von Geflügel, als Futtermittelzusatz Verwendung findet. Dieses MHA kann statt Methionin verwendet werden und verbessert wie dieses beispielsweise die Ausbeute an Brustfleisch bei Geflügel. Darüber hinaus findet es in Form seines Calciumsalzes bei Behandlung der Nierensuffizienz auch pharmazeutische Verwendung.

Eingesetzt wird MHA meist in Form wässriger Konzentrate, wobei diese neben dem Monomeren noch einen gewissen Anteil an Oligomeren, hauptsächlich die di- und trimeren linearen Estersäuren enthalten. Der Gehalt an diesen Oligomeren hängt von den Herstellungsbedingungen und der gewählten Konzentration ab.

Es ist allgemein bekannt, dass 2-Hydroxy-4-methylthiobuttersäure durch Hydratisierung und sukzessive Hydrolyse von 2-Hydroxy-4-methylthiobutyronitril im schwefelsauren Medium kontinuierlich oder batchweise hergestellt werden kann.

Die Synthese wird beispielsweise gemäß EP- A-0 874 811, aber nicht ausschließlich wie folgt durchgeführt:

Das allgemeine Verfahren zur Herstellung von MHA geht von 3-Methylthiopropionaldehyd, auch als Methylmercaptopropionaldehyd oder MMP bezeichnet, aus, der mit Cyanwasserstoff zum 2-Hydroxy-4-methylthiobutyronitril, auch als MMP-Cyanhydrin oder MMP-CH bezeichnet, umgesetzt wird (Gleichung I).

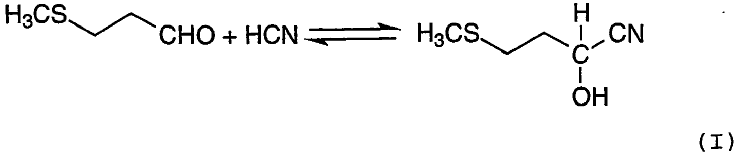

Das entstandene MMP-Cyanhydrin wird anschließend üblicherweise mit starken Mineralsäuren wie Schwefelsäure oder Salzsäure über die Zwischenstufe des 2-Hydroxy-4-methylthiobutyramids, auch als MHA-Amid bezeichnet (Gleichung II) 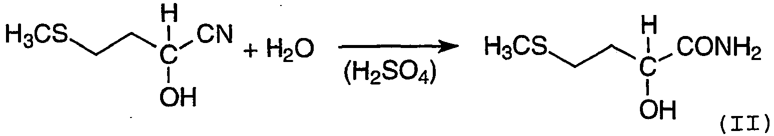 zum Methioninhydroxyanalogen (MHA) hydrolisiert (Gleichung III).

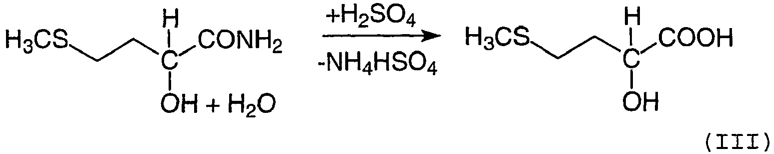

Diese Hydrolyse kann sowohl ein- als auch zweistufig durchgeführt werden, wobei unter "Stufen" zu verstehen ist, dass zur Hydrolyse des MMP-CH's entweder einmal oder zweimal Mineralsäuren und/oder Wasser zugesetzt wird.

So wird beispielsweise die Hydrolyse des MMP-CH so geführt, dass in einer ersten Stufe das MMP-CH mit 60-85 gew.-%iger, bevorzugt mit 65-80 gew.-%iger Schwefelsäure im Molverhältnis MMP-CH zu H₂SO₄ von 1,0:0,5 bis 1:1,0, vorzugsweise 1:0,6 bis 1:0,95 bei Temperaturen von 30-90°C, vorzugsweise 50-70°C zur Herstellung von MHA-Amid hydrolisiert wird. Hierbei entsteht aus dem MMP-Cyanhydrin das MHA-Amid, wobei die entstehende Mischung weiterhin im wesentlichen frei von nicht-umgesetzten MMP-Cyanhydrin ist. Die Hydrolyse verläuft nahezu quantitativ. Das MHA-Amid wird in einer zweiten Stufe unter Zugabe von Wasser ohne weitere Zugabe von H₂SO₄ (es wird beispielsweise eine Schwefelsäurekonzentration von <40% eingestellt) bei Temperaturen bis 140°C, bevorzugt ≤ 110°C hydrolisiert.

Es werden darüber hinaus weitere Verfahren beschrieben, die sich in der Regel lediglich durch den Downstream-Prozess unterscheiden. So wird in der Schrift JP-B-7-97970 die Extraktion von MHA aus der Reaktionsmischung mit Methylisobutylketon beschrieben. EP-A-863 135 beschreibt ein anderes Verfahren mit Zugabe von Ammoniumbisulfat zur Hydrolyselösung. Nach der Reaktion wird ein mit Wasser nicht mischbares, organisches Lösungsmittel zugegeben, mit dem Ergebnis, dass sich in der organischen Phase das MHA anreichert. Es ist weiterhin beschrieben, dass die wässrige Phase mit einem wassermischbaren organischen Lösungsmittel versetzt werden kann, um das entsprechende Ammoniumsulfat auszufällen.

Weitere Verfahren zur Gewinnung von MHA ohne den Einsatz eines organischen Extraktionslösungsmittel, sind beispielsweise in US 4,912,257 oder EP-A-1 149 073 beschrieben. Bei allen diesen Verfahren liegen unterschiedliche Medien komplexer Zusammensetzung mit unterschiedlichen pH-Werten und entsprechende Korrosivität vor. Diese Korrosivität bewirkt, dass jährlich erhebliche Investitionsmittel für die Instandsetzung infolge von falscher Werkstoffauswahl bei oben genannten Prozess erforderlich sind.

Üblicherweise fertigt man die für Produktionsprozesse in korrosiven komplexen Medien benötigten Apparate und Rohrleitungen aus emaillierten Bauteilen, die sich ausgewiesenermaßen durch hohe Korrosionsbeständigkeit für saure Medien auszeichnen. Emaillierte Bauteile besitzen den Nachteil, dass sie gegenüber mechanischen Belastungen, wie sie besonders bei der Montage oder dem täglichen Produktionsbetrieb auftreten, sehr empfindlich sind. Ist die emaillierte Oberfläche erst einmal beschädigt, ist die Korrosion nicht mehr aufzuhalten, da das Trägermaterial in der Regel aus Normalstahl besteht, welcher bekanntermaßen gegenüber sauren Medien wenig beständig ist.

Ein weiterer Nachteil emaillierter Bauteile besteht darin, dass sie in gesonderten Produktionsstätten langwierig vorgefertigt werden müssen und häufig nicht in den gewünschten Abmessungen zur Verfügung stehen. Dies kann unter Umständen zu langen Anlagenstillständen und daraus resultierenden ökonomischen Verlusten führen. Aufgrund der schlechten Wärmeübergangskoeffizienten von emaillierten Bauteilen müssen beispielsweise entsprechende Wärmetauscher großvolumiger gefertigt werden. Dies schlägt sich ebenso in höheren Investitionskosten nieder.

Ein weiterer Nachteil emaillierter Bauteile stellt aufgrund der begrenzten Fertigungsmöglichkeiten die daraus resultierende hohe Anzahl erforderlicher Flanschverbindungen dar, die als potentielle Leckagemöglichkeiten erhöhte Aufwendungen für Umweltschutzmaßnahmen erforderlich machen. Ein weiterer Nachteil des Werkstoffes Emaille ist darin zu sehen, dass aufgrund fertigungstechnischer Hindernisse, konstruktive Freiheitsgrade bei der Fertigung von speziellen ressourcenschonenden Apparaten stark limitiert sind.

Zwar ist beispielsweise für einen kontinuierlichen Prozess zur Herstellung von 2-Hydroxy-4-methylthiobuttersäure in der Patentanmeldung WO96/40630 beschrieben, dass bei den eingesetzten Reaktionsapparaten, Pumpen und Wärmetauscher auf korrosionsresistente Werkstoffe zu achten ist, aber eine Angabe um welche es sich hierbei handelt fehlt.

In der Dechema-Werkstoff-Tabelle (1969, 1971) werden für schwefelsaure Medien austenitische Stähle empfohlen, aber in den für die Reaktion benötigten Konzentrationen und Temperaturen als nicht beständig eingestuft. Die angegebenen Abtragungsraten sind im Hinblick auf die eventuelle daraus resultierenden Schwermetallbelastungen für das in die Nahrungskette gelangende Endprodukt MHA nicht zu tolerieren. Die Korrosionsbeständigkeit von Werkstoffen wird generell z.B. durch Messung der Abtragungsraten in mm p.a. als Maßstab für die erfolgte Korrosion (Definition in Römpp's Lexikon der Chemie, 1990, Seite 2344) ermittelt.

Im vorliegenden Zusammenhang, als korrosionsbeständig anzusehen sind Werkstoffe mit Abtragungsraten mindestens kleiner als 0,06 mm p.a. bei Temperaturen von ≤60°C, oder kleiner als 0,10 mm p.a. bei Temperaturen von ≤110°C

Auch wurde in der Dechema-Werkstoff-Tabelle (1969) im Kapitel Schwefelsäure, Blatt 17, beschrieben, dass durch Zulegieren von Kupfer die Korrosionsbeständigkeit erheblich erhöht werden kann, aber die Abtragungsraten weiterhin unbefriedigend hoch sind. Es besteht für NiCrMoCu-Legierungen eine sogenannte Beständigkeitslücke zwischen 31-82% wässrige Schwefelsäure und Temperaturen >20°C. Die Abtragungsraten liegen beispielsweise bei 80°C und Schwefelsäure Konzentrationen zwischen 10 und 78% bei ca. 0,1 mm per annum (p.a.).

Auch ist nach H. Zitter, Werkstoffe und Korrosion 7 (1957), 758 bekannt, dass Stähle der Zusammensetzung 18% Cr, 22% Ni, 3% Mo und 2% Kupfer im Konzentrationsbereich von 60 bis 80% Schwefelsäure, bei 60°C Abtragungsraten von bis zu 1,8 mm p.a. aufweisen. Weiterhin ist aus der Dechema-Werkstoff-Tabelle bekannt, dass Nickel-Legierungen in schwefelsauren Medien korrosionsresistent sind. So wird beispielsweise für Hastelloy F (48% Ni, 22% Cr, 15% Fe, 6,5% Mo, 2% NS+Ta) eine gute Beständigkeit im Konzentrationsbereich von 2-96% bei Raumtemperatur angegeben. Bei Temperaturen von 66 bzw. 80°C sind die für den MHA-Prozess nicht akzeptablen Abtragungsraten von < 8 mm p.a. angegeben. Außerdem wird berichtet, dass Nickel-Molybdän-Chrom-Legierungen vom Typ (NiMo18Cr17W) bei Raumtemperatur allen Schwefelkonzentrationen widerstehen.

Bei 70°C und Schwefelsäurekonzentrationen von < 15% ist der Abtrag noch als günstig zu bezeichnen. Bei höheren Konzentrationen betragen die Abtragungsraten in der Spitze bis zu 0,5-0,75 mm p.a. So beträgt beispielsweise gemäß Nickel-Informationsbüro GmbH, Düsseldorf (1961, Oktober), Seite 36, für die Legierung Hastelloy C (54% Ni, 16% Mo, 16% Cr, 4% W, 4-6% Fe, 0,05-0,07% C) bei 70°C in 40%iger Schwefelsäure die Abtragungsrate 0,2 mm p.a. Als einziger für die oben genannten Reaktionsbedingungen geeigneter Werkstoff mit akzeptablen Korrosionsraten ist Zirkon anzusehen, der sich aber in vielen Anwendungsfällen aus ökonomischer Sicht verbietet. Auch wurden Normalstahl mit Teflon-Beschichtung oder andere mögliche Werkstoffe, aber ohne näher auf diese einzugehen, genannt. In Beispiel 22 der WO96/40630, wird der Einsatz eines Strömungsrohres, gefertigt aus Normalstahl und mit einer Teflon-Beschichtung, beschrieben. Solche Verbundkonstruktionswerkstoffe besitzen die gleichen Nachteile wie Emaille, beispielsweise aber nicht ausschließlich bezogen auf Wärmedurchgangskoeffizienten und/oder Freiheitsgrade bei der Konstruktion/Fertigung.

### Aufgabe der Erfindung

Es ist daher Aufgabe dieser Erfindung, für die jeweiligen Verfahrensstufen des oben beschriebenen Prozesses geeignete Konstruktionswerkstoffe für Maschinen und Apparate bereitzustellen.

### Beschreibung der Erfindung

Es wurden nun für die Herstellung von 2-Hydroxy-4-methylthiobuttersäure Werkstoffe gefunden, die in der Regel die oben genannten Nachteile nicht besitzen. Das heißt, überflüssige Flanschverbindungen können dank anwendbarer Fügetechniken vermieden werden. Das reduziert in hohem Maße die Leckageanfälligkeit und trägt in einem großen Maße zu einem verbesserten Umweltschutz bei. Mechanische Belastungen, wie sie im allgemeinen Produktionsbetrieb und bei der Montage auftreten, führen zu keinem das Korrosionsverhalten beeinflussende Schäden. Der konstruktiven Optimierung von Reaktionsapparaten sind keine Grenzen gesetzt. Dank der niedrigen Wärmedurchgangswiderstände lassen sich beispielsweise Wärmetauscher mit geringem, ressourcenschonenden Bauvolumen fertigen.

Dieser Erfindung ist ein Verfahren zur Herstellung von 2-Hydroxy-4-methylthiobuttersäure, bei dem das durch Blausäureanlagerung an 3-Methylthiopropionaldehyd erhaltene Additionsprodukt 2-Hydroxy-4-methylthiobutyronitril mit Schwefelsäure über das Intermediat 2-Hydroxy-4-methylthiobutyramid umgesetzt wird, dadurch gekennzeichnet, dass die Reaktion 2-Hydroxy-4-methylthiobutyronitril zum 2-Hydroxy-4-methylthiobutyramid und die anschließende Umsetzung zu 2-Hydroxy-4-methylthiobuttersäure in, für die eingesetzten Reaktionsmedien korrosionsbeständigen, aus legiertem Stahlt und/ oder entsprechenden Nickellegierungen gefertigten Reaktionsbehältern durchgeführt wird, und die Konstruktionswerkstoffe der Reaktionsbehälter.

Abtragungsrate von <0,06 mm p.a., vorzugsweise <0,025 mm p.a., besonders bevorzugt <0,015 mm p.a., ganz besonders bevorzugt <0,01 mm p.a. bei Temperaturen ≤60°C, und <0,1 mm p.a., vorzugsweise <0,06 mm p.a., besonders bevorzugt <0,05 mm p.a., ganz besonders bevorzugt <0,035 mm p.a. bei Temperaturen ≤110°C aufweisen gemessen gemäβ ASTM G4-68, wobei die Reaktion 2-Hydroxy-4-methylthiobutyronitril zu 2-Hydroxy-4-methylthiobutyramid bei Temperaturen ≤ 60°C in Reaktoren, Wärmetauscher, Pumpen und Rohrleitungen, die aus einem Werkstoff ausgewählt aus der Gruppe von 2.4602, 2.4605, 2.4856 und 1.4562 gefertigt wurden, durchgeführt wird.

Im Gegensatz zur oben beschrieben Literatur, wurde überraschender Weise gefunden, dass Legierungen des Typs beispielsweise XNiMoCu oder NiMoCrW unter den oben genannten Prozessbedingungen eingesetzt werden können. Es wurde überraschend gefunden, dass Legierungen des Typs X₁NiMoCu wie z.B. X₁NiCrMoCu 32287 (1.4562) und NiMoCrW wie z.B. NiCr 31Mo14W (2.4602) oder NiMo16Cr15W (Hastelloy C-246) unter den beim MHA-Prozess herrschenden Bedingungen akzeptable Abtragungsraten aufweisen. Legierungen des Typs X₁NiMoCuN beispielsweise der Typ X₁NiMoCuN 25205 (1.4539) bzw. X1NiCrMoCu 31274 (1.4563) sind allerdings nicht geeignet. Darüber hinaus hat sich der Werkstoff vom Typ NiCr23Mo16A1 (2.4605) bzw. NiCr22Mo9Nb (2.4856) überraschenderweise als geeignet herausgestellt.

Im einzelnen wurden folgende Werkstoffe für die entsprechenden Verfahrensschritte gefunden:

Für die Verfahrensstufe, Umsetzung von 2-Hydroxy-4-mehtylthiobutyronitril zu 2-Hydroxy-4-methylthiobutyramid eignen sich als Konstruktionswerkstoffe für Temperaturen ≤60°C, für Reaktoren, Wärmetauscher, Pumpen und Rohrleitungen die Werkstoffe 2.4602, 2.4605, 2.4856, 1.4562.

Die Klassifizierung der Legierungen wurde gemäß DIN EN 10027-2 von 1992 vorgenommen.

Für die sogenannte Hydrolysestufe von 2-Hydroxy-4-methylthiobutyramid zur 2-Hydroxy-4-methylthiobuttersäure bei Temperaturen ≤ 110°C wurde für die Konstruktion der Reaktoren und Wärmetauscher nur Werkstoff 2.4605 als geeignet gefunden. Für die Rohrleitungen eignen sich die Werkstoffe 2.4602 und 2.4605 . Als Konstruktionswerkstoff für Pumpen wurde gefunden, dass sich die Werkstoffe 2.4819 (Hastelloy C-276) und 2.4605 eignen.

Die vorliegende Erfindung wird im folgenden anhand von Ausführungsbeispielen näher erläutert. Diese dienen nur zur Veranschaulichung der Erfindung und sind in keinem Fall als Limitierung in Art und Umfang dieser zu sehen.

### Beispiel 1

Der Reaktionsbehälter, in dem das 2-Hydroxy-4-methylthiobutyronitril in Gegenwart von 65-70-%iger Schwefelsäure bei 50-60°C zu 2-Hydroxy-4-methylthiobutyramid umgesetzt wird, wurde mit Werkstoffproben vom Typ 1.4562 bestückt und nach 250 Stunden begutachtet. Es wurde eine Abtragungsrate von < 0,01 mm p.a. ermittelt.

### Beispiel 2

Es wurde gemäß Beispiel 1 verfahren, jedoch eine Werkstoffprobe vom Typ 2.4605 eingesetzt. Es wurde eine Abtragungsrate von < 0,01 mm p.a. ermittelt.

### Beispiel 3

Der Reaktionsbehälter, in dem das 2-Hydroxy-4-methylthiobutyramid durch Wasserzugabe (Schwefelsäurekonzentration <40%) bei 110°C zu 2-Hydroxy-4-methylthiobuttersäure umgesetzt wird, wurde mit Werkstoffproben vom Typ 2.4602 bestückt und nach 250 Stunden begutachtet. Es wurde eine Abtragungsrate von 0,031 mm p.a. ermittelt.

### Beispiel 4

Es wurde gemäß Beispiel 3 verfahren, jedoch wurde eine Werkstoffprobe vom Typ 2.4605 500 Stunden den Reaktionsbedingungen ausgesetzt. Es wurde eine Abtragungsrate von 0,02 mm p.a. ermittelt.

### Beispiel 5

Es wurde gemäß Beispiel 1 verfahren, jedoch wurde eine Werkstoffprobe vom Typ 2.4856 eingesetzt. Es wurde eine Abtragungsrate von < 0,01 mm p.a. ermittelt.

### Vergleichsbeispiel A

Es wurde gemäß Beispiel 4 verfahren, jedoch wurde eine Werkstoffprobe vom Typ Emaille WWG911 (Lieferant Fa. Pfaudler, Pfaudler Strasse D-68723 Schwetzingen) eingesetzt. Es wurde eine Abtragungsrate von 0,021 mm p.a. ermittelt.

### Vergleichsbeispiel B

Es wurde gemäß Vergleichsbeispiel A verfahren. Eingesetzt wurde ein Emaille 3009 (Lieferant Fa. DeDietrich, Niederbronn / Frankreich). Die Abtragungsrate betrug 0,033 mm p.a..

### Vergleichsbeispiel C

Es wurden Reaktionsbedingungen wie bei Beispiel 1 gewählt, jedoch wurde ein Werkstoff vom Typ 1.4539 eingesetzt. Die Abtragungsrate betrug 0,069 mm p.a.

### Vergleichsbeispiel D

Es wurde gemäß Beispiel 1 verfahren, jedoch wurde ein Werkstoff vom Typ 1.4563 eingesetzt. Es wurde eine Abtragungsrate von 0,06 mm p.a. ermittelt.

### Vergleichsbeispiel E

Es wurde gemäß Beispiel 3 verfahren, jedoch wurde ein Werkstoff vom Typ 1.4562 für 250 Stunden den Reaktionsbedingungen ausgesetzt. Es wurde eine Abtragungsrate von 0,37 mm p.a. ermittelt.

Vorgenannte Untersuchungen erfolgten gemäß ASTM G4-68. Dabei wurden gebeizte Coupons (60 x 20 mm) aus dem betreffenden Werkstoff im Reaktionsmedium eingesetzt. Die Beizung wurde bei Raumtemperatur für 1 bis 2 Stunden durchgeführt. Als Beizlösung wurde 24 Vol.% wässrige HF (40%), 8 Vol.% wässrige HNO₃ (65%) in Wasser verwendet.

Nach Ablauf der Testzeit wurden die Proben nacheinander mit Wasser und Aceton gewaschen und anschließend mit einem Heißluftgebläse bis zur Gewichtskonstanz getrocknet.

Der durch Korrosion bedingte Gewichtsverlust wurde dann durch Wägung und Vergleich mit dem ursprünglichen Gewicht des Coupons ermittelt. Aus dem Gewichtsverlust wurde die Abtragungsrate in mm p.a. berechnet.

Eine Zusammenfassung aller untersuchten Werkstoffe, deren Zusammensetzung ist in Tabelle 1 gegeben. Die Ergebnisse der Beispiele 1- 5, sowie Vergleichsbeispiele A-E sind in Tabelle 2 zusammengefasst.

**Tabelle 1**

| **Werkstoffe zum Einsatz im MHA-Process (Angaben zur Zusammensetzung in %)** | | | | | | | | | | | | |
|---|---|---|---|---|---|---|---|---|---|---|---|---|
| **Werkstoff Nr** | **Kurzname** | **Fe** | **Cr** | **Ni** | **Mo** | **Cu** | **Al** | **N** | **W** | **Nb** | **Mn** | **Co** |
| 1.4539 | X1NiCrMoCuN 25 20 5 | Rest | 19-21 | 24-25 | 4-5 | 1-2 | ---- | 0,04-0,15 | --- | --- | --- | --- |
| 1.4562 | X1NiCrMoCu 32 28 7 | Rest | 26-28 | 30-32 | 6-7 | 1-1,4 | --- | 0,15-0,25 | --- | --- | --- | --- |
| 1.4563 | X1NiCrMoCu 31 27 4 | Rest | 26-28 | 30-32 | 3-4 | 0,8- 1,5 | --- | --- | --- | --- | <=2 | --- |
| 2.4602 | NiCr21Mo14W | 2-6 | 20-22,5 | Rest | 12,5-14,5 | --- | --- | --- | 2,5-3,5 | --- | --- | < 2,5 |
| 2.4605 | NiCr23Mo16A1 | max 1,5 | 22-24 | Rest | 15-16,5 | --- | 0,1-0,4 | --- | --- | --- | max 0,5 | max 0,3 |
| 2.4819 | NiMo16Cr15W (Hastelloy C-276) | 4-7 | 15-16,5 | Rest | 15-17 | --- | --- | --- | 3-4,5 | --- | --- | < 2,5 |
| 2.4856 | NiCr22Mo9Nb | 4 | 22 | Rest | 9 | --- | --- | --- | --- | 3,8 | --- | --- |

**Tabelle 2**

| **Beispiel / Vergleichsbsp.** | **Werkstoff** | **Temperatur, °C** | **Einsatzdauer, (h)** | **Abtragungsra te, mm p.a.** | **Eignung des Werkstoffes** |
|---|---|---|---|---|---|
| 1 | 1.4562 | 50-60 | 250 | <0,01 | ja |
| 2 | 2.4605 | 50-60 | 250 | <0,01 | ja |
| 3 | 2.4602 | 110 | 250 | 0,031 | ja |
| 4 | 2.4605 | 110 | 500 | 0,02 | ja |
| 5 | 2.4856 | 50-60 | 250 | <0,01 | ja |
| A | Emaille WWG911 | 110 | 500 | 0,021 | ja |
| B | Emaille 3009 | 110 | 500 | 0,033 | ja |
| c | 1.4539 | 50-60 | 250 | 0,069 | nein |
| D | 1.4563 | 50-60 | 250 | 0,06 | nein |
| E | 1.4562 | 110 | 250 | 0,37 | nein |

## Patentansprüche

1. Verfahren zur Herstellung von 2-Hydroxy-4-methylthiobuttersäure, bei dem das durch Blausäureanlagerung an 3-Methylthiopropionaldehyd erhaltene Additionsprodukt 2-Hydroxy-4-methylthiobutyronitril mit Schwefelsäure über das Intermediat 2-Hydroxy-4-methylthiobutyramid umgesetzt wird, **dadurch gekennzeichnet, dass** die Reaktion 2-Hydroxy-4-methylthiobutyronitril zum 2-Hydroxy-4-methylthiobutyramid und die anschließende Umsetzung zu 2-Hydroxy-4-methylthiobuttersäure in, für die eingesetzten Reaktionsmedien korrosionsbeständigen, aus legiertem Stahl und/oder Nickellegierungen gefertigten Reaktionsbehältern durchgeführt wird, und die Konstruktionswerkstoffe der Reaktionsbehälter Abtragsraten von < 0,06 mm p. a. bei Temperaturen von ≤ 60°C oder von < 0,1 mm p. a. bei Temperaturen von ≤ 110°C aufweisen gemessen gemäß ASTM G4-68, wobei die Reaktion 2-Hydroxy-4-methylthiobutyronitril zu 2-Hydroxy-4-methylthiobutyramid bei Temperaturen ≤ 60°C in Reaktoren, Wärmetauscher, Pumpen und Rohrleitungen, die aus einem Werkstoff ausgewählt aus der Gruppe von 2.4602, 2.4605, 2.4856 und 1.4562 gefertigt wurden, durchgeführt wird.

2. Verfahren gemäß Anspruch 1, **dadurch gekennzeichnet, dass** die Reaktoren, Wärmetauscher, Pumpen und Rohrleitungen aus dem Werkstoff 2.4602 gefertigt wurden.

3. Verfahren gemäß Anspruch 1, **dadurch gekennzeichnet, dass** die Reaktoren, Wärmetauscher, Pumpen und Rohrleitungen aus dem Werkstoff 2.4605 gefertigt wurden.

4. Verfahren gemäß Anspruch 1, **dadurch gekennzeichnet, dass** die Reaktoren, Wärmetauscher, Pumpen und Rohrleitungen aus dem Werkstoff 2.4856 gefertigt sind.

5. Verfahren gemäß Anspruch 1, **dadurch gekennzeichnet, dass** die Reaktoren, Wärmetauscher, Pumpen und Rohrleitungen aus dem Werkstoff 1.4562 gefertigt sind.

6. Verfahren gemäß Anspruch 1, **dadurch gekennzeichnet, dass** die Hydrolysestufe von 2-Hydroxy-4-methylthiobutyramid zur 2-Hydroxy-4-methylthiobuttersäure bei Temperaturen ≤ 110°C in Reaktoren und Wärmetauschern aus Werkstoff 2.4605 durchgeführt wird.

7. Verfahren gemäß Anspruch 1, **dadurch gekennzeichnet, dass** die Hydrolysestufe von 2-Hydroxy-4-methylthiobutyramid zur 2-Hydroxy-4-methylthiobuttersäure bei Temperaturen ≤ 110°C mit Rohrleitungen aus den Werkstoffen 2.4602 oder 2.4605 durchgeführt wird.

8. Verfahren gemäß Anspruch 7, **dadurch gekennzeichnet, dass** das Verfahren mit Rohrleitungen aus dem Werkstoff 2.4605 durchgeführt wird.

9. Verfahren gemäß Anspruch 1, **dadurch gekennzeichnet, dass** die Hydrolysestufe von 2-Hydroxy-4-methylthiobutyramid zur 2-Hydroxy-4-methylthiobuttersäure bei Temperaturen ≤ 110°C mit Pumpen aus den Werkstoffen 2.4819 (Hastelloy C-276) oder 2.4605 durchgeführt wird.

10. Verfahren gemäß Anspruch 9, **dadurch gekennzeichnet, dass** das Verfahren mit Pumpen aus dem Werkstoff 2.4819 durchgeführt wird.

## Claims

1. Process for preparing 2-hydroxy-4-methylthio-butyric acid, in which the 2-hydroxy-4-methylthio-butyronitrile addition product obtained by addition of hydrogen cyanide onto 3-methylthio-propionaldehyde is reacted with sulphuric acid via the 2-hydroxy-4-methylthiobutyramide intermediate, **characterized in that** the reaction of 2-hydroxy-4-methylthiobutyronitrile to give 2-hydroxy-4-methylthiobutyramide and the subsequent conversion to 2-hydroxy-4-methylthiobutyric acid are performed in reaction vessels which are corrosion-resistant to the reaction media used and are manufactured from alloyed steel and/or nickel alloys, and the construction materials of the reaction vessels have material removal rates of < 0.06 mm p.a. at temperatures of ≤ 60°C or of < 0.1 mm p.a. at temperatures of ≤ 110°C, measured according to ASTM G4-68, the reaction of 2-hydroxy-4-methylthiobutyronitrile to give 2-hydroxy-4-methylthiobutyramide being performed at temperatures of ≤ 60°C in reactors, heat exchangers, pumps and pipelines which have been manufactured from a material selected from the group of 2.4602, 2.4605, 2.4856 and 1.4562.

2. Process according to Claim 1, **characterized in that** the reactors, heat exchangers, pumps and pipelines have been manufactured from the material 2.4602.

3. Process according to Claim 1, **characterized in that** the reactors, heat exchangers, pumps and pipelines have been manufactured from the material 2.4605.

4. Process according to Claim 1, **characterized in that** the reactors, heat exchangers, pumps and pipelines have been manufactured from the material 2.4856.

5. Process according to Claim 1, **characterized in that** the reactors, heat exchangers, pumps and pipelines have been manufactured from the material 1.4562.

6. Process according to Claim 1, **characterized in that** the hydrolysis stage of 2-hydroxy-4-methyl-thiobutyramide to give 2-hydroxy-4-methylthio-butyric acid is performed at temperatures of ≤ 110°C in reactors and heat exchangers made of material 2.4605.

7. Process according to Claim 1, **characterized in that** the hydrolysis stage of 2-hydroxy-4-methyl-thiobutyramide to give 2-hydroxy-4-methylthio-butyric acid is performed at temperatures of ≤ 110°C in pipelines made of materials 2.4602 or 2.4605.

8. Process according to Claim 7, **characterized in that** the process is performed with pipelines made of material 2.4605.

9. Process according to Claim 1, **characterized in that** the hydrolysis stage of 2-hydroxy-4-methyl-thiobutyramide to give 2-hydroxy-4-methylthio-butyric acid is performed at temperatures of ≤ 110°C with pumps made of materials 2.4819 (Hastelloy C-276) or 2.4605.

10. Process according to Claim 9, **characterized in that** the process is performed with pumps made of material 2.4819.

## Revendications

1. Procédé de préparation d'acide 2-hydroxy-4-méthylthiobutyrique, dans lequel le produit d'addition, le 2-hydroxy-4-méthylthiobutyronitrile, obtenu par addition d'acide cyanhydrique sur du 3-méthylthiopropionaldéhyde est transformé avec de l'acide sulfurique via l'intermédiaire 2-hydroxy-4-méthylthiobutyramide, **caractérisé en ce que** la réaction du 2-hydroxy-4-méthylthiobutyronitrile en 2-hydroxy-4-méthylthiobutyramide et la transformation consécutive en acide 2-hydroxy-4-méthylthiobutyrique sont réalisées dans des récipients de réaction fabriqués en alliage d'acier et/ou en alliages de nickel, résistants à la corrosion pour les milieux de réaction utilisés, et les matériaux de construction des récipients de réaction présentent des pertes de matière < 0,06 mm par an à des températures ≤ 60°C ou < 0,1 mm par an à des températures ≤ 110°C, mesurées selon la norme ASTM G4-68, la réaction de 2-hydroxy-4-méthylthiobutyronitrile en 2-hydroxy-4-méthylthiobutyramide étant réalisée à des températures ≤ 60°C dans des réacteurs, des échangeurs de chaleur, des pompes et des conduites fabriqués en un matériau choisi dans le groupe formé par les matériaux 2.4602, 2.4605, 2.4856 et 1.4562.

2. Procédé selon la revendication 1, **caractérisé en ce que** les réacteurs, les échangeurs de chaleur, les pompes et les conduites ont été fabriqués en matériau 2.4602.

3. Procédé selon la revendication 1, **caractérisé en ce que** les réacteurs, les échangeurs de chaleur, les pompes et les conduites ont été fabriqués en matériau 2.4605.

4. Procédé selon la revendication 1, **caractérisé en ce que** les réacteurs, les échangeurs de chaleur, les pompes et les conduites sont fabriqués en matériau 2.4856.

5. Procédé selon la revendication 1, **caractérisé en ce que** les réacteurs, les échangeurs de chaleur, les pompes et les conduites sont fabriqués en matériau 1.4562.

6. Procédé selon la revendication 1, **caractérisé en ce que** l'étape d'hydrolyse de 2-hydroxy-4-méthylthiobutyramide en acide 2-hydroxy-4-méthylthiobutyrique est réalisée à des températures ≤ 110°C dans des réacteurs et des échangeurs de chaleur en matériau 2.4605.

7. Procédé selon la revendication 1, **caractérisé en ce que** l'étape d'hydrolyse de 2-hydroxy-4-méthylthiobutyramide en acide 2-hydroxy-4-méthylthiobutyrique est réalisée à des températures ≤ 110°C avec des conduites en matériaux 2.4602 ou 2.4605.

8. Procédé selon la revendication 7, **caractérisé en ce que** le procédé est réalisé avec des conduites en matériau 2.4605.

9. Procédé selon la revendication 1, **caractérisé en ce que** l'étape d'hydrolyse de 2-hydroxy-4-méthylthiobutyramide en acide 2-hydroxy-4-méthylthiobutyrique est réalisée à des températures ≤ 110°C avec des pompes en matériaux 2.4819 (Hastelloy C-276) ou 2.4605.

10. Procédé selon la revendication 9, **caractérisé en ce que** le procédé est réalisé avec des pompes en matériau 2.4819.
